# EUROPEAN PATENT APPLICATION

(11) **EP 4 726 026 A1**
(43) Date of publication of application: **15.04.2026**
(21) Application number: 25817125.5
(22) Date of filing: 13.05.2025
(51) Int. Cl.: C12N 1/20, A61K 35/747, A61K 35/745, A61K 9/08, A61K 9/19, A61P 1/00, C12R 1/23, C12R 1/01

(54) **PROBIOTIC COMPOSITION FOR PREVENTING AND TREATING INTESTINAL INFLAMMATION AND USE THEREOF**

(30) Priority: 14.08.2024 CN 202411110938
(71) Applicant: Wecare Probiotics Co., Ltd., Suzhou, Jiangsu 215200 (CN)
(72) Inventor: FANG, Shuguang, Suzhou, Jiangsu 215200 (CN); SI, Wenjin, Suzhou, Jiangsu 215200 (CN); DONG, Yao, Suzhou, Jiangsu 215200 (CN); GAI, Zhonghui, Suzhou, Jiangsu 215200 (CN); ZHU, Jianguo, Suzhou, Jiangsu 215200 (CN)
(74) Representative: Buzzi, Notaro & Antonielli d'Oulx S.p.A.
(86) International application number: PCT/CN2025/094613
(87) International publication number: WO 2026/036811

(57) **Abstract**

Provided are a probiotic composition for preventing and treating an intestinal inflammation and a use thereof. The probiotic composition for preventing and treating an intestinal inflammation includes a *Lactobacillus acidophilus* LA85 strain and a *Bifidobacterium longum* subsp. *longum* BL36 strain. A brand-new probiotic compounding manner and a brand-new strategy for preventing and treating an intestinal inflammation are creatively developed. It is found that the LA85 strain and the BL36 strain can cooperate, promote each other and exert a synergistic effect in preventing and treating an intestinal inflammation, which is specifically manifested by: decreasing the concentrations of proinflammatory cytokines TNF-α, IL-6 and IL-1β in mouse serum, and increasing the concentration of anti-inflammatory cytokines IL-10; increasing the concentration of urolithin A in mouse serum, and significantly decreasing the concentration of reactive oxygen species in serum; and significantly improving the levels of Zonula Occludens-1, tight junction proteins and Claudin-1 of intestinal barrier indicators in mouse serum.

## Description

### TECHNICAL FIELD

The present application belongs to the technical field of microorganisms and relates to a probiotic composition for preventing and treating an intestinal inflammation and a use thereof.

### BACKGROUND

The intestinal tract, which is the body's first line of defense, is not only responsible for the selective absorption of nutritive substances, but also responsible for resisting the invasion of inflammatory factors and harmful microorganisms. When the body is in a compromised state or suddenly exposed to a large number of pathogens and virulence factors, an intestinal inflammation and an intestinal mucosal injury are caused, further resulting in systemic inflammatory response syndrome and multiple organ failure. Lipopolysaccharide (LPS), which is one of the stressors that induce an intestinal mucosal injury, can not only damage the intestinal tract, but also cause bacterial translocation. The damaged gastrointestinal barrier causes the abnormal expression of intestinal tight junction proteins, allowing endotoxin and pathogens to pass through the intestinal wall. Toll-like receptors 4 (TLR4s) on the cell surfaces are induced and activated to stimulate the downstream nuclear factor-κB (NF-κB) signaling pathway, and inflammatory factors of cells are synthesized and released, thereby aggravating an intestinal injury.

Probiotics, especially Bifidobacterium and Lactobacillus, are known for their ability to improve intestinal health and regulate immunity. On the one hand, probiotics can improve the intestinal inflammation by alleviating the existing intestinal injury, restoring the damaged intestinal barrier and enhancing the immune system. On the other hand, probiotics can increase the level of tight junction (TJ) proteins between intestinal epithelial cells. Probiotics can also inhibit the nuclear factor-κB (NF-κB) signaling pathway, down-regulate proinflammatory cytokines and up-regulate anti-inflammatory cytokines, thereby maintaining the immune balance and protecting the immune barrier.

It can be seen that probiotics has an effect of regulating intestinal health. Probiotics can alleviate the intestinal inflammation by promoting intestinal barrier functions, regulating inflammatory markers and regulating the balance of immune cells. Further, probiotics neither promote the spread of antibiotic resistance nor carry transferable antibiotic resistance. Therefore, probiotics are safe, green and harmless alternative products to antibiotics. It is very meaningful to find and develop more probiotic intervention strategies, especially probiotic combination strategies, for preventing and treating intestinal inflammations.

### SUMMARY

The present application provides a probiotic composition for preventing and treating an intestinal inflammation and a use thereof.

In a first aspect, the present application provides a probiotic composition for preventing and treating an intestinal inflammation. The probiotic composition for prevent and treating an intestinal inflammation includes a *Lactobacillus acidophilus* LA85 strain with a deposit number CGMCC No. 1.12735 and a *Bifidobacterium longum* subsp. *longum* BL36 strain with a deposit number CGMCC No. 24413.

The *Lactobacillus acidophilus* LA85 strain is deposited in the China General Microbiological Culture Collection Center on July 20, 2020, with a deposit number CGMCC No. 1.12735, where the China General Microbiological Culture Collection Center is located at No. 3, NO. 1 West Beichen Road, Chaoyang District, Beijing.

The *Bifidobacterium longum* subsp. *longum* BL36 strain is deposited in the China General Microbiological Culture Collection Center on February 21, 2022, with a deposit number CGMCC No. 24413, where the China General Microbiological Culture Collection Center is located at No. 3, NO. 1 West Beichen Road, Chaoyang District, Beijing.

The present application creatively develops a brand-new probiotic compounding manner and a brand-new strategy for preventing and treating an intestinal inflammation, that is, the *Lactobacillus acidophilus* LA85 strain and the *Bifidobacterium longum* subsp. *longum* BL36 strain are compounded. It is found that the two strains can cooperate, promote each other and exert a synergistic effect in preventing and treating an intestinal inflammation, which is specifically manifested by: (1) decreasing the concentrations of proinflammatory cytokines TNF-α, IL-6 and IL-1β in mouse serum, and increasing the concentration of anti-inflammatory cytokines IL-10; (2) increasing the concentration of urolithin A in mouse serum, and significantly decreasing the concentration of reactive oxygen species in serum; and (3) significantly improving the levels of Zonula Occludens-1, tight junction proteins and Claudin-1 of intestinal barrier indicators in mouse serum.

In the case where amounts of bacteria used are consistent, compared with an intervention mode of a single LA85 strain or a single BL36 strain, the compounding of the two bacteria significantly improves the above effect. Therefore, the probiotic composition has a good prospect of being used for preparing the product for preventing, alleviating or treating an intestinal inflammation. Moreover, the two bacteria are both probiotics, and the product has high safety and is not easy to develop resistance.

Preferably, a ratio of viable bacterial count of the LA85 strain to the viable bacterial count of the BL36 strain is 1:10 to 10:1, for example, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1 or 10:1. Other specific point values within the numerical range may be selected, and details are not described here.

In a second aspect, the present application provides a probiotic preparation for preventing and treating an intestinal inflammation. The probiotic preparation includes the probiotic composition of the first aspect.

Preferably, a total viable bacterial count in the probiotic preparation is not less than 1×10⁹ CFU/mL or 1×10⁹ CFU/g, for example, 1×10⁹ CFU/g (CFU/mL), 2×10⁹ CFU/g (CFU/mL), 5×10⁹ CFU/g (CFU/mL), 8×10⁹ CFU/g (CFU/mL), 1×10¹⁰ CFU/g (CFU/mL), 5×10¹⁰ CFU/g (CFU/mL), 1×10¹¹ CFU/g (CFU/mL), 1×10¹² CFU/g (CFU/mL) or 1×10¹³ CFU/g (CFU/mL). Other specific point values within the numerical range may be selected, and details are not described here.

Preferably, a dosage form of the probiotic preparation includes a solution, lyophilized powder, a capsule, a tablet or granules.

Preferably, the dosage form of the probiotic preparation is the solution, and the solution is prepared through the following method:
inoculating a LA85 strain and a BL36 strain in mediums, respectively, for activation and fermentation culture to obtain fermentation broths; separately centrifuging the fermentation broths, and separately resuspending fermentation broths with a solvent to obtain a LA85 bacterial suspension and a BL36 bacterial suspension; and mixing the LA85 bacterial suspension with the BL36 bacterial suspension according to a ratio of viable bacterial counts to obtain the probiotic preparation.

Preferably, the dosage form of the probiotic preparation is the lyophilized powder, and the lyophilized powder is prepared through the following method:
inoculating a LA85 strain and a BL36 strain in mediums, respectively, for activation and fermentation culture to obtain fermentation broths; separately centrifuging the fermentation broths, separately mixing the fermentation broths with a protective agent, and freeze drying the mixtures to obtain LA85 bacteria powder and BL36 bacteria powder; and mixing the LA85 bacteria powder with the BL36 bacteria powder according to a ratio of viable bacterial counts to obtain the probiotic preparation.

In a third aspect, the present application provides a use of the probiotic composition of the first aspect or the probiotic preparation of the second aspect for preparing a drug for preventing, alleviating or treating an intestinal inflammation.

Preferably, the drug further includes an adjuvant.

Preferably, the adjuvant is selected from any one or a combination of at least two of a filler, a binder, a wetting agent, a disintegrating agent, an emulsifier, a cosolvent, a solubilizer, an osmotic pressure regulator, a colorant, a pH adjusting agent, an anti-oxidant, a bacteriostatic agent or a buffer.

Compared with the prior art, the present application has the beneficial effects described below.

The present application creatively develops a brand-new probiotic compounding manner and a brand-new strategy for preventing and treating an intestinal inflammation, that is, the *Lactobacillus acidophilus* LA85 strain and the *Bifidobacterium longum* subsp. *longum* BL36 strain are compounded. It is found that the two strains can cooperate, promote each other and exert a synergistic effect in preventing and treating an intestinal inflammation, which is specifically manifested by: (1) decreasing the concentrations of proinflammatory cytokines TNF-α, IL-6 and IL-1β in mouse serum, and increasing the concentration of anti-inflammatory cytokines IL-10; (2) increasing the concentration of urolithin A in mouse serum, and significantly decreasing the concentration of reactive oxygen species in serum; and (3) significantly improving the levels of Zonula Occludens-1, tight junction proteins and Claudin-1 of intestinal barrier indicators in mouse serum.

In the case where amounts of bacteria used are consistent, compared with an intervention mode of a single LA85 strain or a single BL36 strain, the compounding of the two bacteria significantly improves the above effect. Therefore, the probiotic composition has a good prospect of being used for preparing the product for preventing, alleviating or treating an intestinal inflammation. Moreover, the two bacteria are both probiotics, and the product has high safety and is not easy to develop resistance.

### BRIEF DESCRIPTION OF DRAWINGS

FIG. 1 is a chart illustrating the statistical result of the level of interleukin-1β (IL-1β) in serum of each group of mice.
FIG. 2 is a chart illustrating the statistical result of the level of interleukin 6 (IL-6) in serum of each group of mice.
FIG. 3 is a chart illustrating the statistical result of the level of interleukin 10 (IL-10) in serum of each group of mice.
FIG. 4 is a chart illustrating the statistical result of the level of tumor necrosis factor α (TNF-α) in serum of each group of mice.
FIG. 5 is a chart illustrating the statistical result of the level of reactive oxygen species (ROS) in serum of each group of mice.
FIG. 6 is a chart illustrating the statistical result of the level of urolithin A in serum of each group of mice.
FIG. 7 is a chart illustrating the statistical result of the level of Zonula Occludens-1 (ZO-1) in serum of each group of mice.
FIG. 8 is a chart illustrating the statistical result of the level of tight junction proteins (Occludin) in serum of each group of mice.
FIG. 9 is a chart illustrating the statistical result of the level of Claudin-1 in serum of each group of mice.

### DETAILED DESCRIPTION

To further elaborate on the technical means adopted and the effects achieved in the present application, the solutions of the present application are further described below with reference to the preferable examples of the present application, but the present application is not limited to the scope of the examples.

The medium involved in the following example is as follows:
MRS medium (g/L): 10 g/L peptone, 10 g/L beef extract, 15 g/L glucose, 15 g/L lactose, 5 g/L yeast powder, 2 g/L diammonium hydrogen citrate, 2.6 g/L K₂PO₄·3H₂O, 0.1 g/L MgSO₄·7H₂O, 0.05 g/L MnSO₄, 1 mL/L Tween 80 and 0.5 g/L cysteine salt.

The taxonomic name of the LA85 strain involved in the following example is *Lactobacillus acidophilus* deposited on July 20, 2020, with a deposit number CGMCC No. 1.12735.

The taxonomic name of the BL36 strain involved in the present application is *Bifidobacterium longum* subsp. *longum* deposited on February 21, 2022, with a deposit number CGMCC No. 24413.

The bacterial suspension involved in the following example: required strains were inoculated in an MRS liquid medium and cultured for 24 h at 37°C for activation to obtain an activation solution, the activation solution was inoculated in an MRS liquid medium at an inoculum volume of 3% (v/v) and cultured for 24 h at 37°C to obtain a bacterial solution, the bacterial solution was centrifuged at 6000 g for 6 min, and the strains were resuspended with PBS to obtain the bacterial suspension.

### Example

This example explores the ability of a probiotic composition to alleviate symptoms of an intestinal inflammation model mouse.
(1) Test animals: male C57BL/6 mice (6-week-old) (70 mice, purchased from Shanghai Laboratory Animal Center). These mice were reared in a controlled environment with room temperature maintained at 22±2°C and a humidity of 55%±5%, following a 12 h light/dark cycle. They had free access to food and water. All experimental procedures involving the mice were in accordance with the ethical guidelines for animal care and use established by Shanghai Laboratory Animal Care and Experimental Center.
(2) Animal grouping: after the above mice were adaptively fed for 1 week, the 70 mice were randomly divided into 7 groups (10 mice for each group): a blank control group (CON), a model group (LPS), a LA85 group (LA85), a BL36 group (BL36), a composite probiotic group 1 (a LA85+BL36 group with a ratio of viable bacterial counts of 1:1), a composite probiotic group 2 (a LA85+ATCC15707 group with a ratio of viable bacterial counts of 1:1) and a composite probiotic group 3 (an ATCC4356+BL36 group with a ratio of viable bacterial counts of 1:1).
(3) Animal modeling and intervention methods

On Day 1 to Day 21 of the formal experiment, the blank control group and the model group were fed normally+normal purified water, and each probiotic intervention group was fed normally+probiotic drinking water (dosage of probiotics: 10⁹ CFU/mouse/day). On Day 22 of the formal experiment, each mouse in the model group and the probiotic intervention groups were intraperitoneally injected with 1.5 mg/kg LPS, and samples were taken 24 h after the injection.

### (4) Sampling

After the intervention, the mice were sacrificed, and blood was collected from eyeballs. After centrifugation at 1200×g for 10 min, serum was extracted and stored at -80°C.

### (5) Indicator analysis

### (5.1) Detection of inflammatory factor indicators

Levels of interleukin-1β (IL-1β), interleukin 6 (IL-6), interleukin 10 (IL-10) and tumor necrosis factor α (TNF-α) in serum of mice in each group were detected by using a mouse inflammatory factor ELISA scientific research kit.

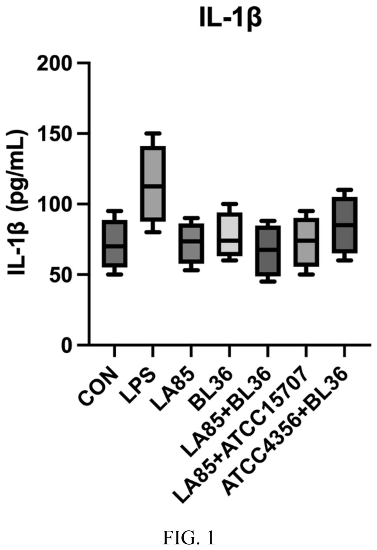

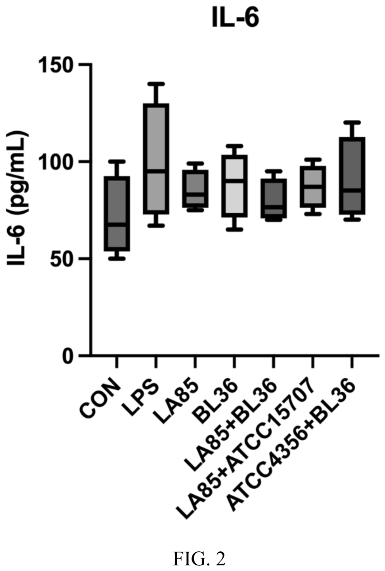

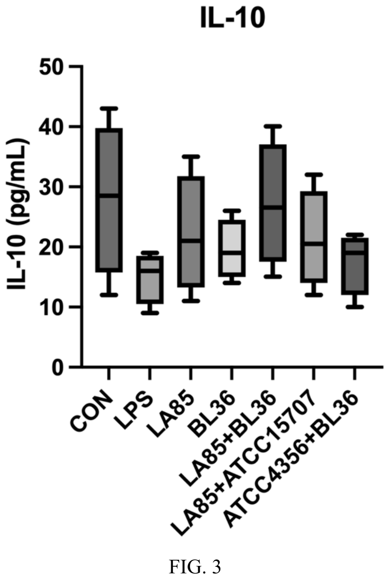

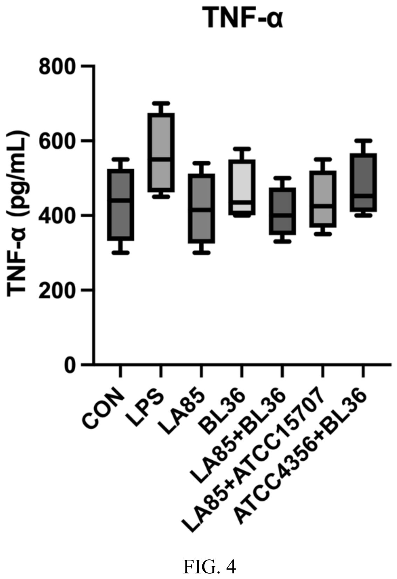

The results are shown in FIGS. 1 to 4, respectively. Compared with the blank control group, the mice in the model group have significantly increased levels of TNF-α, IL-1β and IL-6 and a significantly decreased level of IL-10. After the intervention of the probiotics, the probiotic intervention groups can produce different degrees of reversal effects, and a better reversal effect of the composite probiotic group 1 can significantly reduce the levels of the proinflammatory factors TNF-α, IL-1β and IL-6 of the mice and significantly increase the level of the anti-inflammatory factor IL-10, making the levels of the inflammatory factors of the serum of the mice closer to normal levels. It indicates that the probiotic composition involved in the present application has a good effect of preventing and treating an intestinal inflammation.

### (5.2) Detection of antioxidant factors

Levels of reactive oxygen species (ROS) and urolithin A in the serum of the mice were detected by using a mouse antioxidant factor ELISA scientific research kit.

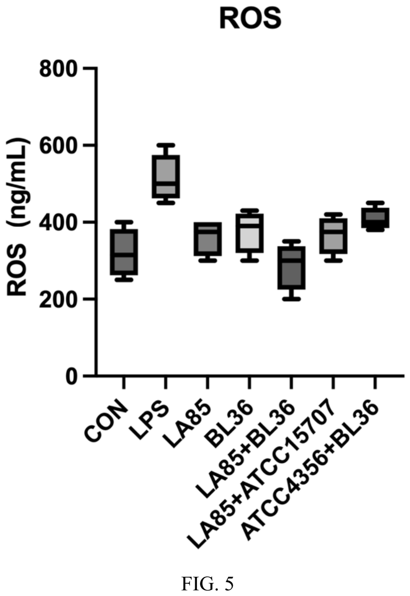

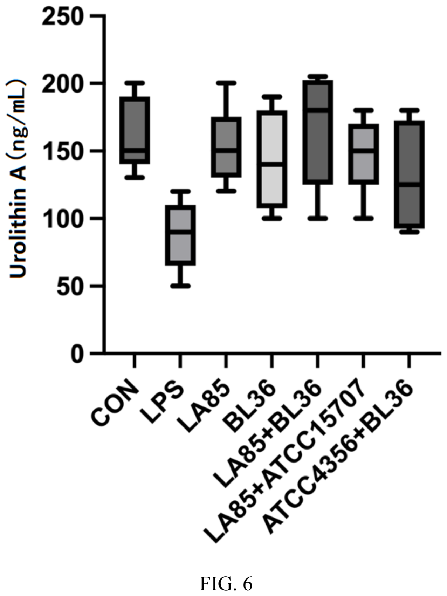

The results are shown in FIGS. 5 and 6, respectively. Compared with the blank control group, the serum of the mice in the model group has an increased concentration of ROS and a significantly decreased concentration of urolithin A. After the intervention of the probiotics, the concentration of ROS is significantly decreased, and the concentration of urolithin A is significantly increased. The composite probiotic group 1 has the most significant effect. It indicates that the probiotic composition involved in the present application has a good effect of preventing and treating an intestinal inflammation.

### (5.3) Detection of intestinal barrier indicators

Levels of Zonula Occludens-1 (ZO-1), tight junction proteins (Occludin) and Claudin-1 in the serum of the mice were detected by using a mouse intestinal barrier indicator ELISA scientific research kit.

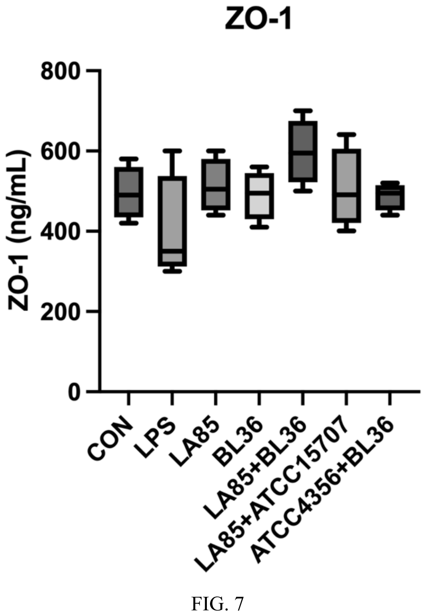

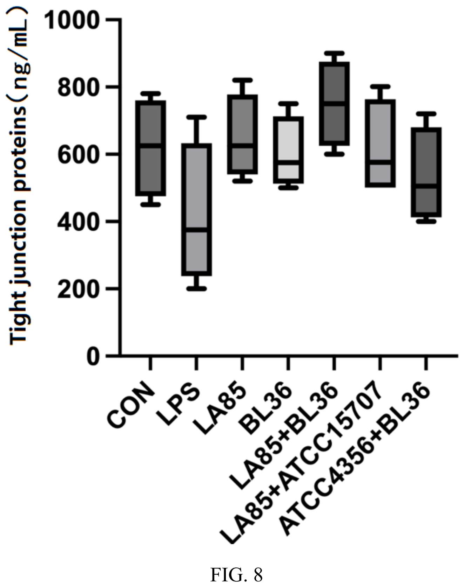

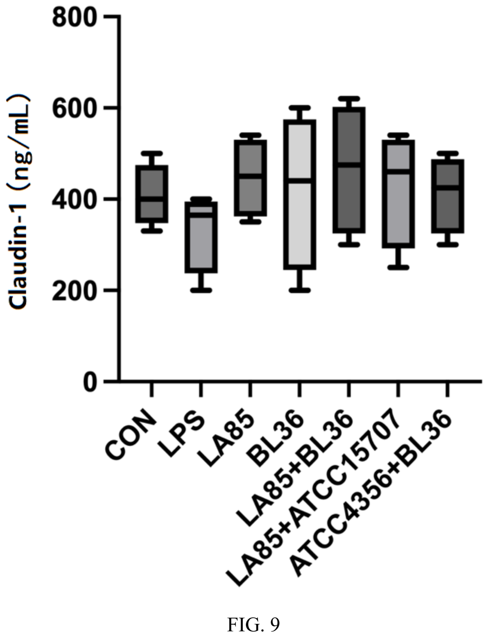

The results are shown in FIGS. 7 to 9, respectively. Compared with the blank control group, the serum of the mice in the model group has significantly decreased levels of Zonula Occludens-1 (ZO-1), tight junction proteins (Occludin) and Claudin-1, indicating that the epithelial barrier integrity of the mice is impaired and the intestinal permeability of the mice is increased. After the intervention of the probiotics, different degrees of reversal effects can be produced, and a better reversal effect of the composite probiotic group 1 can significantly improve the levels of Zonula Occludens-1 (ZO-1), tight junction proteins (Occludin) and Claudin-1 of the intestinal barrier indicators of the mice. It indicates that the probiotic composition involved in the present application has a good effect of preventing and treating an intestinal inflammation.

The applicant has stated that although the technical solutions of the present application are described through the preceding embodiments, the present application is not limited to the preceding embodiments, which means that the implementation of the present application does not necessarily depend on the preceding embodiments. It is to be understood by those skilled in the art that any improvements made to the present application and equivalent replacements of raw materials of the product, additions of adjuvant ingredients, selections of specific manners, etc. in the present application all fall within the scope of the present application.

Although preferred embodiments of the present application are described above in detail, the present application is not limited to details of the preceding embodiments, and various simple variations can be made to the technical solutions of the present application without departing from the technical concept of the present application. These simple variations are all within the scope of the present application.

Additionally, it is to be noted that if not in collision, specific technical features described in the preceding embodiments can be combined in any suitable manner. To avoid unnecessary repetition, various possible combinations are no longer described in the present application.

## Claims

1. A probiotic composition for preventing and treating an intestinal inflammation, comprising a *Lactobacillus acidophilus* LA85 strain with a deposit number CGMCC No. 1.12735 and a *Bifidobacterium longum* subsp. *longum* BL36 strain with a deposit number CGMCC No. 24413.

2. The probiotic composition for preventing and treating an intestinal inflammation according to claim 1, wherein a ratio of viable bacterial count of the LA85 strain to viable bacterial count of the BL36 strain is 1:10 to 10:1.

3. A probiotic preparation for preventing and treating an intestinal inflammation, comprising the probiotic composition according to claim 1 or 2.

4. The probiotic preparation according to claim 3, wherein a total viable bacterial count in the probiotic preparation is not less than 1×10⁹ CFU/mL or 1×10⁹ CFU/g.

5. The probiotic preparation according to claim 3, wherein a dosage form of the probiotic preparation comprises a solution, lyophilized powder, a capsule, a tablet or granules.

6. The probiotic preparation according to claim 5, wherein the dosage form of the probiotic preparation is the solution, and the solution is prepared through the following method:
inoculating a LA85 strain and a BL36 strain in mediums, respectively, for activation and fermentation culture to obtain fermentation broths; separately centrifuging the fermentation broths, and separately resuspending fermentation broths with a solvent to obtain a LA85 bacterial suspension and a BL36 bacterial suspension; and mixing the LA85 bacterial suspension with the BL36 bacterial suspension according to a ratio of viable bacterial counts to obtain the probiotic preparation.

7. The probiotic preparation according to claim 5, wherein the dosage form of the probiotic preparation is the lyophilized powder, and the lyophilized powder is prepared through the following method:
inoculating a LA85 strain and a BL36 strain in mediums, respectively, for activation and fermentation culture to obtain fermentation broths; separately centrifuging the fermentation broths, separately mixing the fermentation broths with a protective agent, and freeze drying the mixtures to obtain LA85 bacteria powder and BL36 bacteria powder; and mixing the LA85 bacteria powder with the BL36 bacteria powder according to a ratio of viable bacterial counts to obtain the probiotic preparation.

8. A use of the probiotic composition according to claim 1 or 2 or the probiotic preparation according to any one of claims 3 to 7 for preparing a drug for preventing, alleviating or treating an intestinal inflammation.

9. The use according to claim 8, wherein the drug further comprises an adjuvant.

10. The use according to claim 9, the adjuvant is selected from any one or a combination of at least two of a filler, a binder, a wetting agent, a disintegrating agent, an emulsifier, a cosolvent, a solubilizer, an osmotic pressure regulator, a colorant, a pH adjusting agent, an anti-oxidant, a bacteriostatic agent or a buffer.
